# EUROPEAN PATENT APPLICATION

(11) **EP 4 368 152 A2**
(43) Date of publication of application: **15.05.2024**
(21) Application number: 23204145.9
(22) Date of filing: 17.10.2023
(51) Int. Cl.: A61F 2/966, A61F 2/07, A61F 2/06

(54) **PROXIMAL STENT-GRAFT RETENTION SYSTEM**

(30) Priority: 20.10.2022 US 202263380256 P; 01.09.2023 US 202318459808
(71) Applicant: Medtronic Vascular Inc., Santa Rosa, CA 95403 (US)
(72) Inventor: FLORY, David Eron, Santa Rosa (US); BAZOR, Benjamin, Santa Rosa (US); BHANDAL, Vincent De Leon, Santa Rosa (US); GODIN, Andrew, Santa Rosa (US); PATEL, Manthan P., Santa Rosa (US)
(74) Representative: Zimmermann & Partner Patentanwälte mbB

(57) **Abstract**

A proximal stent-graft retention system includes a stent-graft having a suture loop attached to a proximal end of the stent-graft and a delivery catheter having a distal tip that includes a retention feature. The retention feature can include a slot extending through a sidewall of the distal tip and a trigger wire. The slot of the distal tip exposes a portion of the trigger wire within the tip lumen such that the trigger wire may extend through the suture loop of the stent-graft at the slot of the distal tip of the delivery catheter and is configured to retain the proximal end of the stent-graft in the radially expanded configuration via the suture loop to prevent distal migration and compression of the stent-graft. Proximal retraction of the trigger wire releases the suture loop of the stent-graft such that the stent-graft is no longer coupled to the delivery catheter.

## Description

### CROSS REFERENCE OF RELATED APPLICATIONS

This application claims the benefit of U.S. Provisional Patent Application No. 63/380256, filed October 20, 2022, which is hereby incorporated by reference in its entirety for all purposes.

### FIELD OF THE INVENTION

The present invention relates to a delivery system for medical devices. More particularly, the present invention relates to a delivery system having a retention system configured to be used in conjunction with a stent-graft.

### BACKGROUND

An aneurysm is an excessive localized enlargement of an artery caused by a weakening of the artery wall. Treatment options for aneurysms vary depending on the location, size and condition of the aneurysm. A common treatment method involves deploying a stent-graft within the diseased artery to direct blood flow through the aneurysm and protecting the artery wall to prevent the aneurysm from bursting. A conventional stent-graft typically includes a radially expandable reinforcement structure, e.g., formed from a stent or a plurality of annular stent rings, and a cylindrically shaped layer of graft material defining a lumen to which the stent/stent rings are coupled.

Aneurysms may extend into the iliac arteries (also referred to as the common iliac arteries), which branch from the aorta. Each common iliac artery branches into an external iliac artery and an internal iliac artery, also referred to as the hypogastric artery. Procedures currently use multiple stent-grafts, deployed one at a time, to treat such aneurysms. There are many challenges with such procedures. In most cases, after deployment of a first stent-graft, subsequent stent-grafts must be tracked through the deployed first stent-graft. The tracking of a second delivery system into and through the deployed first stent-graft can compress the proximal section of the first stent-graft, resulting in less length to connect a subsequent stent-graft, which can reduce joint strength and patency. The tracking of the second delivery system can also cause the entire deployed first stent-graft to migrate distally, causing the same issues as proximal compression with the addition of possibly restricting the flow of the subsequent connecting stent-graft in the iliac arteries.

Embodiments hereof relate to improvements configured to prevent proximal compression and/or distal migration of the first deployed stent-graft during subsequent procedural steps.

### BRIEF SUMMARY OF THE INVENTION

In accordance with first example hereof, a retention system includes a stent-graft having a proximal body having a proximal-most stent ring, a graft material, and a loop attached to a proximal end of the stent-graft, wherein the stent-graft has a radially compressed configuration for delivery within a vasculature and a radially expanded configuration for deployment. The retention system further includes a delivery catheter having an inner shaft having a shaft lumen extending from a distal end to a proximal end of the inner shaft, wherein the stent-graft is disposed over a distal portion of the inner shaft in the radially compressed configuration, a distal tip coupled to the distal end of the inner shaft, the distal tip including a tip lumen and a slot extending through a sidewall of the distal tip, wherein the tip lumen is fluidly connected to the shaft lumen to form a trigger wire lumen of the delivery catheter, and a trigger wire extending through the trigger wire lumen of the delivery catheter. The slot of the distal tip exposes a portion of the trigger wire within the tip lumen. The trigger wire extends through the loop of the stent-graft at the slot of the distal tip of the delivery catheter and is configured to retain the proximal end of the stent-graft in the radially expanded configuration via the loop to prevent distal migration and compression of the stent-graft. Proximal retraction of the trigger wire releases the loop of the stent-graft such that the stent-graft is no longer coupled to the delivery catheter.

In a second example, in the retention system of the first example, the stent-graft is a bifurcated stent-graft, the bifurcated stent-graft having an internal branch and an external branch that extend distally from the proximal body.

In a third example, in the retention system of the first example, the loop is attached to the proximal-most stent ring at the proximal end of the stent-graft.

In a fourth example, in the retention system of the first example, the loop is attached to the graft material at the proximal end of the stent-graft.

In a fifth example, in the retention system of the first example, the trigger wire is a guidewire for the delivery catheter.

In a sixth example, in the retention system of the first example, the loop is a single loop such that the proximal end of the stent-graft includes exactly one loop.

In a seventh example, in the retention system of the first example, the loop has a length of about 3-20 mm.

In an eighth example, in the retention system of the first example, the loop is configured to constrain less than 20% of the perimeter of the stent-graft when the stent-graft is in the radially expanded configuration.

In a ninth example, in the retention system of the first example, the proximal-most stent ring of the stent-graft includes a plurality of proximal-most crowns.

In a tenth example, in the retention system of the ninth example, the loop is coupled to a single proximal-most crown of the proximal-most stent ring.

In an eleventh example, in the retention system of the ninth example, the loop is coupled to exactly two proximal-most crowns of the proximal-most stent ring, the exactly two proximal-most crowns being directly adj acent to each other.

In a twelfth example, a retention system includes a stent-graft having a proximal body having a proximal segment including a proximal-most stent ring and a graft material, wherein the stent-graft has a radially compressed configuration for delivery within a vasculature and a radially expanded configuration for deployment. The retention system further includes a delivery catheter having an inner shaft having a shaft lumen extending from a distal end to a proximal end of the inner shaft, and a distal tip coupled to the distal end of the inner shaft, the distal tip including a tip lumen and a distal-facing notch formed in a sidewall of the distal tip, wherein the tip lumen is fluidly connected to the shaft lumen to form a guidewire lumen of the delivery catheter. The distal-facing notch is configured to receive the proximal segment of the stent-graft such that the distal-facing notch retains the proximal end of the stent-graft in the radially expanded configuration to prevent distal migration and compression of the stent-graft. Proximal retraction of the delivery catheter releases the loop from the distal-facing notch of the distal tip such that the stent-graft is no longer coupled to the delivery catheter.

In a thirteenth example, in the retention system of the twelfth example, the stent-graft is a bifurcated stent-graft, the bifurcated stent-graft having an internal branch and an external branch that extend distally from the proximal body.

In a fourteenth example, in the retention system of the twelfth example, the proximal-most stent ring of the stent-graft includes a plurality of proximal-most crowns and the proximal segment of the stent-graft to be received by the distal-facing notch is a single proximal-most crown of the proximal-most stent ring of the stent-graft.

In a fifteenth example, in the retention system of the twelfth example, the proximal segment of the stent-graft to be received by the distal-facing notch is a loop attached to a proximal end of the stent-graft.

In a sixteenth example, in the retention system of the fifteenth example, the loop is attached to the proximal-most stent ring at the proximal end of the stent-graft.

In a seventeenth example, in the retention system of the fifteenth example, the loop is attached to the graft material at the proximal end of the stent-graft.

In an eighteenth example, in the retention system of the fifteenth example, the loop is a single loop such that the proximal end of the stent-graft includes exactly one loop.

In the nineteenth example, in the retention system of the fifteenth example, loop has a length of about 3-20 mm.

In the twentieth example, in the retention system of the fifteenth example, the loop is configured to constrain less than 20% of the perimeter of the stent-graft when the stent-graft is in the radially expanded configuration.

In a twenty-first example, in the retention system of the fifteenth example, the loop is coupled to a single proximal-most crown of the proximal-most stent ring.

In a twenty-second example, in the retention system of the fifteenth example, the loop is coupled to exactly two proximal-most crowns of the proximal-most stent ring, the exactly two proximal-most crowns being directly adj acent to each other.

In a twenty-third example, the distal-facing notch is a protrusion formed by a slit that extends through a portion of the sidewall of the distal tip, the protrusion extending radially outward and in a distal direction towards a distal end of the distal tip.

In a twenty-fourth example, in the retention system of the twenty-third example, a distal surface of the distal-facing notch forms an angle between 30 degrees and 60 degrees relative to a longitudinal axis of the delivery catheter.

In a twenty-fifth example, in the retention system of the twenty-third example, the proximal segment of the stent-graft to be received by the distal-facing notch hooks onto the distally-facing notch and proximal retraction of the delivery catheter results in proximal retraction of the distal-facing notch such that the proximal segment of the stent-graft is no longer hooked onto the distally-facing notch.

In a twenty-sixth example, a method includes loading a first bifurcated stent-graft in a radially compressed configuration onto a first delivery catheter, wherein the first bifurcated stent-graft includes a proximal body having a proximal-most stent ring, an internal branch and an external branch extending distally from the proximal body, a graft material, and a loop coupled to the proximal-most stent ring, and the first delivery catheter including an inner shaft, an outer shaft, and a distal tip having a retention feature, coupling the loop of the first bifurcated stent-graft to the retention feature of the distal tip of the first delivery catheter, tracking the first bifurcated stent-graft and the first delivery catheter through a first external iliac artery within a vasculature via a first guidewire, deploying the proximal body and the internal branch of the first bifurcated stent-graft to a radially expanded configuration, wherein the loop of the first bifurcated stent-graft remains coupled to the retention feature of the distal tip of the first delivery catheter, loading an internal branch extension stent-graft in a radially compressed configuration on a second delivery catheter, tracking the internal branch extension stent-graft and the second delivery catheter through a second external iliac artery, the proximal body and the internal branch of the first bifurcated stent-graft via a second guidewire, deploying the internal branch extension stent-graft within the internal branch of the first bifurcated stent-graft, removing the second delivery catheter from the vasculature, deploying the external branch of the first bifurcated stent-graft to a radially expanded configuration, wherein the loop of the first bifurcated stent-graft remains coupled to the retention feature of the distal tip of the first delivery catheter, releasing the loop of the first bifurcated stent-graft from the retention feature of the distal tip of the first delivery catheter such that the first bifurcated stent-graft is no longer coupled to the first delivery catheter, and removing the first delivery catheter from the vasculature.

In a twenty-seventh example, in the method of the twenty-sixth example, deployment of the proximal body and the internal branch of the first bifurcated stent-graft is achieved via proximal retraction of the outer shaft of the first delivery catheter.

In a twenty-eighth example, in the method of the twenty-sixth example, deployment of the internal branch extension stent-graft is achieved via proximal retraction of an outer shaft of the second delivery catheter.

In a twenty-ninth example, in the method of the twenty-sixth example, deployment of the external branch of the first bifurcated stent-graft is achieved via further proximal retraction of the outer shaft of the first delivery catheter.

In a thirtieth example, in the method of the twenty-sixth example, the method further comprises deploying a second bifurcated stent-graft within an aorta of the vasculature, the second bifurcated stent-graft including a proximal body, a first branch and a second branch extending distally from the proximal body of the second bifurcated stent-graft, deploying a first branch extension stent-graft that extends from the first branch of the second bifurcated stent-graft to the proximal body of the first bifurcated stent-graft, and deploying a second branch extension stent-graft that extends from the second branch of the second bifurcated stent-graft to the second external iliac artery of the vasculature.

In a thirty-first example, in the method of the twenty-sixth example, the loop has a length of about 3-20 mm.

In a thirty-second example, in the method of the twenty-sixth example, the retention feature of the first delivery catheter is configured to prevent distal migration and compression of the first bifurcated stent-graft during delivery and deployment of the internal branch extension stent-graft.

In a thirty-third example, in the method of the twenty-sixth example, the first delivery catheter further includes a trigger wire disposed within a tip lumen of the distal tip.

In a thirty-fourth example, in the method of the thirty-third example, the retention feature is a slot formed in a sidewall of the distal tip that exposes a portion of the trigger wire within the tip lumen, and wherein the trigger wire extends through the loop of the first bifurcated stent-graft at the slot of the distal tip.

In a thirty-fifth example, in the method of the thirty-fourth example, releasing the loop of the first bifurcated stent-graft from the retention feature is achieved via proximal retraction of the trigger wire from the loop.

In a thirty-sixth example, in the method of the thirty-third example, the trigger wire is a guidewire for the first delivery catheter.

In a thirty-seventh example, in the method of the twenty-sixth example, the retention feature of the first delivery catheter is a distal-facing notch formed in a sidewall of the distal tip that is configured to receive and secure the loop or the proximal-most stent ring of the first bifurcated stent-graft.

In a thirty-eighth example, in the method of the thirty-seventh example, the loop of the first bifurcated stent-graft from the retention feature is achieved via proximal retraction of the first delivery catheter.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings may not be to scale.
FIG. 1A shows an idealized example of a patient's vasculature according to an embodiment hereof.
FIG. 1B shows the patient's vasculature of FIG. 1A including an exemplary common iliac aneurysm and an exemplary internal iliac aneurysm according to an embodiment hereof.
FIG. 2 shows a side view of an exemplary stent-graft according to embodiments hereof.
FIG. 2A shows a side view of a proximal end of a stent-graft including a suture loop according to another embodiment hereof.
FIG. 3 shows a side view of a delivery system according to an embodiment hereof, the delivery system including a distal tip with a slot formed in a sidewall thereof.
FIG. 3A shows a cross-sectional view of the delivery system of FIG. 3 taken along line A-A of FIG. 3.
FIG. 3B shows a cross-sectional view of the delivery system of FIG. 3 taken along line B-B of FIG. 3.
FIG. 3C shows a sectional view of the delivery system of FIG. 3 taken along line C-C of FIG. 3.
FIG. 3D shows side view of a retention system of the delivery system of FIG. 3 and a portion of the stent-graft of FIG. 2, the retention system including the slot that receives the suture loop of the stent-graft and a trigger wire, according to embodiments hereof.
FIG. 3E shows the delivery system of FIG. 3 and the stent-graft of FIG. 2 within a vasculature of a patient according to embodiments hereof.
FIG. 3F shows a cross-sectional view taken along line F-F of FIG. 3E.
FIG. 3G shows a sectional view of a distal tip of a delivery system according to another embodiment hereof.
FIG. 3H shows side view of a retention system of the delivery system of FIG. 3G and a portion of the stent-graft of FIG. 2, the retention system including a slot and a guidewire, according to embodiments hereof.
FIG. 4 shows a side view of a delivery system according to another embodiment hereof, the delivery system including a distal tip with a distal-facing notch.
FIG. 4A shows a cross-sectional view of the delivery system of FIG. 4 taken along line A-A of FIG. 4.
FIG. 4B shows a cross-sectional view of the delivery system of FIG. 4 taken along line B-B of FIG. 4.
FIG. 4C shows a sectional view of the delivery system of FIG. 4 taken along line C-C of FIG. 4.
FIG. 4D shows side view of a retention system of the delivery system of FIG. 4 and a portion of the stent-graft of FIG. 2, the retention system including the distal-facing notch that receives the suture loop of the stent-graft, according to embodiments hereof.
FIG. 4E shows side view of a retention system of the delivery system of FIG. 4 and a portion of a stent-graft according to another embodiment hereof, the retention system including the distal-facing notch that receives a crown of the stent-graft, according to embodiments hereof.
FIG. 4F shows side view of a retention system of the delivery system of FIG. 4 and a portion of a stent-graft according to another embodiment hereof, the retention system including a trigger wire and the distal-facing notch that receives a crown of the stent-graft, according to embodiments hereof.
FIG. 5 is a block diagram that shows a method of using a retention system according to embodiments hereof.
FIG. 6 shows an idealized example of a patient's vasculature according to an embodiment hereof.
FIG. 7 shows a step in the method of FIG. 5, showing a first and second guidewire being tracked though the vasculature according to embodiments hereof.
FIG. 8 shows a step in the method of FIG. 5, showing a first delivery system being tracked over the first guidewire according to embodiments hereof.
FIG. 9 shows a step in the method of FIG. 5, showing a first bifurcated stent-graft being partially deployed according to embodiments hereof.
FIG. 10 shows a step in the method of FIG. 5, showing a cross over sheath being tracked over the second guidewire according to embodiments hereof.
FIG. 11 shows a step in the method of FIG. 5, showing a third guidewire being tracked through the cross over sheath and into an internal iliac artery of the patient according to embodiments hereof.
FIG. 12 shows a step in the method of FIG. 5, showing the placement of the third guidewire after retraction of the cross over sheath.
FIG. 13 shows a step in the method of FIG. 5, showing an internal branch extension stent-graft being deployed according to embodiments hereof.
FIG. 14 shows a step in the method of FIG. 5, showing the first bifurcated stent-graft being removed from a retention feature of the first delivery system and removing the first delivery system from the vasculature according to embodiments hereof.
FIG. 15 shows a step in the method of FIG. 5, showing a second bifurcated stent-graft being deployed according to embodiments hereof.
FIG. 16 shows a step in the method of FIG. 5, showing a first branch extension stent-graft being deployed according to embodiments hereof.
FIG. 17 shows in the method of FIG. 5, showing a second branch extension stent-graft being deployed according to embodiments hereof.

### DETAILED DESCRIPTION

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a delivery device. The following detailed description is merely exemplary in nature and is not intended to limit the invention of the application and uses of the invention. Furthermore, there is no intention to be bound by any expressed or implied theory presented in the preceding field of the invention, background, summary or the following detailed description.

As used in this specification, the singular forms "a," "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%. It should be understood that use of the term "about" also includes the specifically recited number of value.

With regard to a delivery system, the terms "proximal" and "distal" herein are used with reference to the clinician. Therefore, "proximal" and "proximally" mean in the direction toward the clinician, and "distal" and "distally" mean in the direction away from the clinician. With regard to a stent-graft prosthesis, the terms "proximal" and "distal" herein are used with reference to proximity of the heart. Therefore, "proximal" and proximally" mean in the direction towards the heart, and "distal" and "distally" mean in the direction away from the heart.

Further, numerical terms such as "first," "second," "third," etc. used herein are not meant to be limiting such that use of the term "second" when referring to a part in the specification does not mean that there necessarily is a "first" of part in order to fall within the scope of the invention. Instead, such numbers are merely describing that the particular embodiment being described has a "first" part and a "second" part. The invention is instead defined by the claims, in which one or more of the numbered parts may be claimed.

Embodiments hereof relate to a retention system for securing a proximal end of a stent-graft to a delivery system during a procedure. The retention system includes a loop, hereinafter referred to as a suture loop, non-removably secured to the proximal end of the stent-graft and a retention feature on a tip of the delivery system that is configured to retain the suture loop of the stent-graft. While referred to as a suture loop, the loop may be formed of any suitable material, such as a polymer, metal, or natural/organic material. The retention system is configured to prevent migration and/or compression of the stent-graft during a procedure. More particularly, the retention system can be used to effectively couple the distal end of the delivery system to the proximal end of the first deployed stent-graft to maintain the position of the first deployed stent-graft until the procedure is finished.

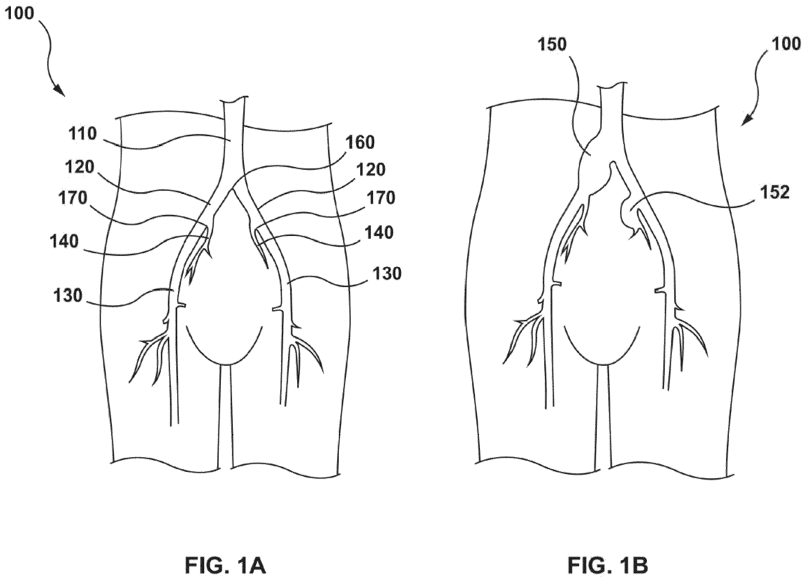

FIGS. 1A-1B illustrate an exemplary patient's vasculature 100 that may be treated with a retention system described below according to embodiments herein. The vasculature 100 includes an aorta 110 that runs vertically along the trunk or body of the patient. The aorta 110 splits at a first bifurcation 160, located in an abdominal region of the patient, into two common iliac arteries 120 that run down each of the patient's legs. Each common iliac artery 120 splits into an external iliac artery 130 and an internal iliac artery 140 at a second bifurcation 170, as shown in FIG. 1A. The internal iliac artery 140 is the smaller terminal branch of the common iliac artery 120. The internal iliac arteries 140 supply blood to the majority of the pelvis and the structures therewithin, while the external iliac arteries 130 continue down the leg to supply blood to the lower extremities. FIG. 1B shows an exemplary common iliac aneurysm 150 and an exemplary internal iliac aneurysm 152 within the vasculature 100 of the patient. The common iliac aneurysm 150 can develop within the common iliac arteries 120 and the internal iliac aneurysm 152 can develop within the internal iliac arteries 140 of the vasculature 100.

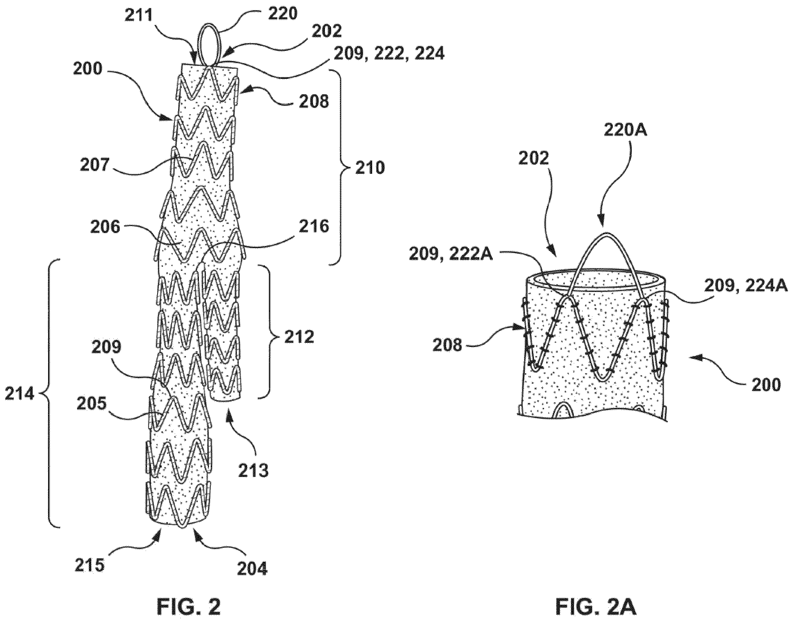

FIG. 2 shows an exemplary stent-graft 200 in a radially expanded or deployed configuration according to embodiments herein. In the embodiment shown, the stent-graft 200 is a bifurcated stent-graft 200 and more particularly, the stent-graft 200 is an asymmetrical bifurcated stent-graft 200. The bifurcated stent-graft 200 has a proximal end 202 and a distal end 204. The bifurcated stent-graft includes a graft material 206 and one or more stent rings 207 attached to an outer surface of the graft material 206. In further embodiments, the one or more stent rings 207 are attached to an inner surface of the graft material 206. In the embodiment shown, the stent rings 207 include a plurality of individual, spaced apart stent rings 207 which are generally cylindrical. For description purposes only, the stent ring 207 that is coupled adjacent to the proximal end 202 of the bifurcated stent-graft 200 is referred to herein as the proximal-most stent ring 208. Each stent ring 207 may be self-expanding structures, e.g., formed of nickel titanium alloy (nitinol) or other shaped memory material, or they may be balloon-expandable stents. Each stent ring 207 is a sinusoidal patterned ring including a plurality of crowns or bends 209 and a plurality of struts or straight segments 205 with each crown 209 being formed between a pair of opposing struts 205. Although shown with fifteen stent rings 207, it will be understood by one of ordinary skill in the art that the bifurcated stent-graft 200 may include a greater or smaller number of stent rings 207 depending upon the desired length of the bifurcated stent-graft 200 and/or the intended application thereof. Stent rings 207 are shown in FIG. 2 as having identical sinusoidal patterns but it will be understood by one of ordinary skill in the art that one or more of stent rings 207 may have a different pattern or configuration.

In FIG. 2, the scaffolding or support of the stent-graft prostheses has been illustrated as a series of independent or separate self-expanding stents/sinusoidal patterned rings. However, as will be understood by one of ordinary skill in the art, the support structure or scaffolding of a stent-graft prosthesis may have other configurations such as a series of sinusoidal patterned rings coupled to each other to form a self-expanding stent. In another embodiment, the support structure or scaffolding of a stent-graft prosthesis may be a unitary tubular component.

The graft material 206 may be any suitable graft material known to be utilized in vascular grafts. For example, the graft material 206 may be a non-permeable material, e.g., a polyester terephthalate (PET), expanded polyester terephthalate (ePET), or polytetrafluoroethylene (PTFE) based material, or other non-permeable graft material.

As shown in FIG. 2, the bifurcated stent-graft 200 includes a proximal body 210 adjacent to the proximal end 202 of the bifurcated stent-graft 200. The proximal body 210 includes a proximal body lumen 211, defined by the inner surface of the graft material 206, that has a substantially circular-shaped cross-section. The proximal body 210 may be configured to be deployed, for example, within a common iliac artery 120 of a patient's vasculature 100. The bifurcated stent-graft 200 further includes an internal branch 212 and an external branch 214 that extend distally from the proximal body 210 of the bifurcated stent-graft 200. The internal branch 212 includes an internal branch lumen 213 and the external branch 214 includes an external branch lumen 215. Each of the internal and external branch lumens 213, 215 have a substantially circular-shaped cross-section. The internal and external branches 212, 214 of the bifurcated stent-graft 200 can articulate or move independently of one another along substantially their entire length, except at a point of bifurcation 216, e.g., where the proximal body 210 splits into the internal branch 212 and the external branch 214. The internal and external branches 212, 214 of the bifurcated stent-graft 200 extend in a direction generally parallel to the proximal body 210 such that their longitudinal axes may be generally parallel. The proximal body 210 of the bifurcated stent-graft 200 can have a longitudinal length ranging from 30-60 mm and the proximal body lumen 211 can have a diameter ranging from 12-20 mm. The internal branch 212 of the bifurcated stent-graft 200 can have a longitudinal length ranging from 20-60 mm and the internal branch lumen 213 can have a diameter ranging from 6-12 mm. The external branch 214 of the bifurcated stent-graft 200 can have a longitudinal length ranging from 40-80 mm and the external branch lumen 215 can have a diameter ranging from 8-16 mm.

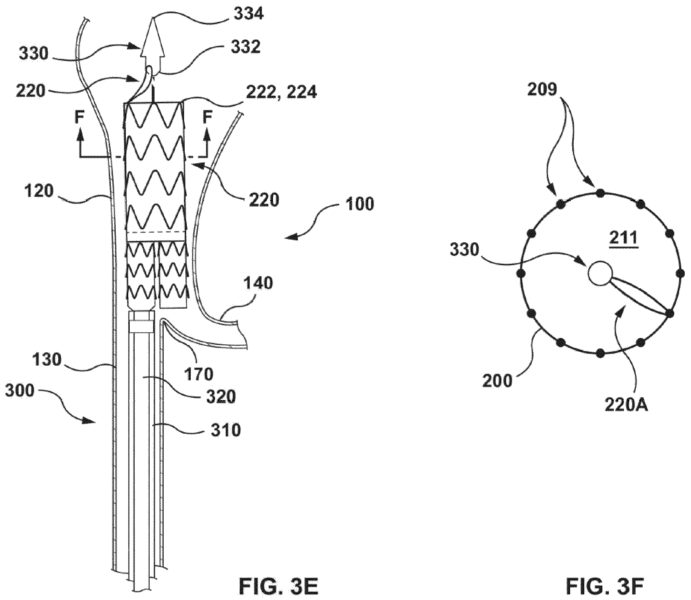

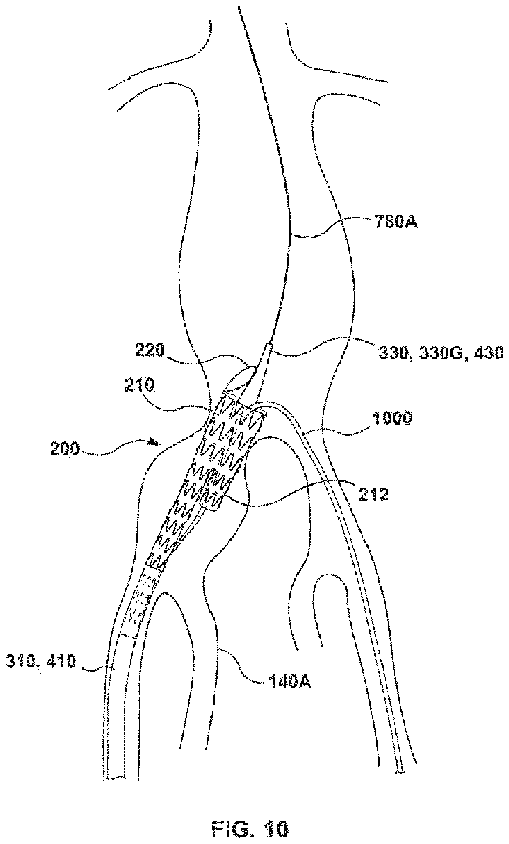

The stent-graft 200 further includes a suture loop 220, which is configured to engage a retention feature of a delivery system as will be described in more detail herein. The suture loop 220 is a strand or segment of suture or other material that includes a first end 222 and a second end 224. In the embodiments shown, the suture loop 220 is coupled to the proximal-most stent ring 208 at the proximal end 202 of the stent-graft 200. In one embodiment, the suture loop 220 can be coupled to a single crown 209 of the proximal-most stent ring 208, as shown in FIG. 2. In such an embodiment, both the first and second ends 222, 224 of the suture are secured to the same crown 209 of the proximal-most stent ring 208, creating or forming a generally circular suture loop 220 having a singular attachment point to the stent-graft 200. The suture loop 220 extends proximally beyond the proximal end 202 of the stent-graft 200. The circumferential position of suture loop 220 in FIG. 2 is exemplary and may vary from the position shown in FIG. 2. For example, as shown in FIG. 3E and FIG. 10 herein, in an embodiment the suture loop 220 is oriented on the proximal end 202 of the first bifurcated stent-graft 200, on a side opposite of the internal branch 212 of the bifurcated stent-graft 200. As shown in and described with respect to FIG. 10, the suture loop 220 is preferably circumferentially positioned to stabilize the bifurcated stent-graft 200 as a cross over sheath is advanced through the internal branch 212 during a method of use.

The suture loop 220 has a longitudinal length, i.e., from the first and second ends 222, 224 of the suture loop 220 to the turn or bend in the suture loop when the suture loop is fully extended, that can range from about 4-16 mm. As will be described in more detail herein with respect to FIGS. 3E and 3F, the length of the suture loop 220 is configured to engage a retention feature of a delivery system when the proximal end 202 of the stent-graft 200 is fully radially expanded or deployed. Stated another way, the suture loop 220 is configured to permit radial expansion and deployment of the proximal end 202 of the stent-graft 200 while remaining coupled to the retention feature of the delivery system.

Although shown with both the first and second ends 222, 224 of the suture loop 220 secured to the same crown of the stent-graft 200, the suture loop may alternatively have a generally U-shaped configured with the first and second ends each attached to a different crown 209 of the stent-graft 200. For example, as show in FIG. 2A, a suture loop 220A is coupled to exactly two adjacent crowns 209 of the proximal-most stent ring 208. In such an embodiment, a first end 222A of the suture is secured to a first crown 209 of the proximal-most stent ring 208 and a second end 224A of the suture is secured to a second crown 209 of the proximal-most stent ring 208 that is directly adjacent to the first crown 209, creating a suture loop 220A having exactly two attachment points to the stent-graft 200. In another embodiment (now shown), the suture loop 220 can be attached to the graft material 206 of the stent-graft 200 at the proximal end 202 rather than to one or more crowns of the proximal-most stent ring 208.

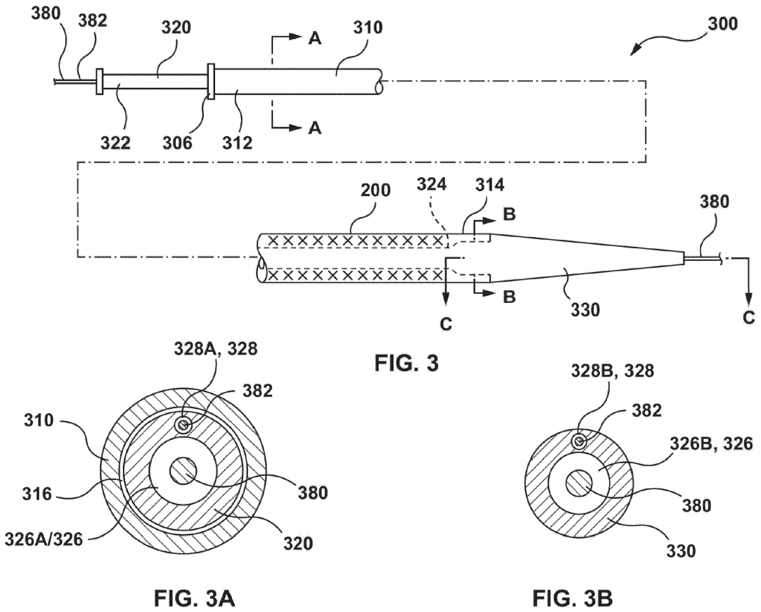

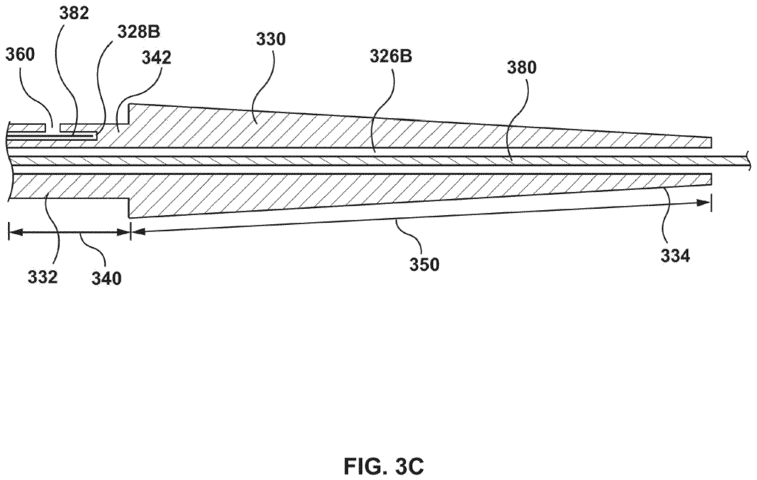

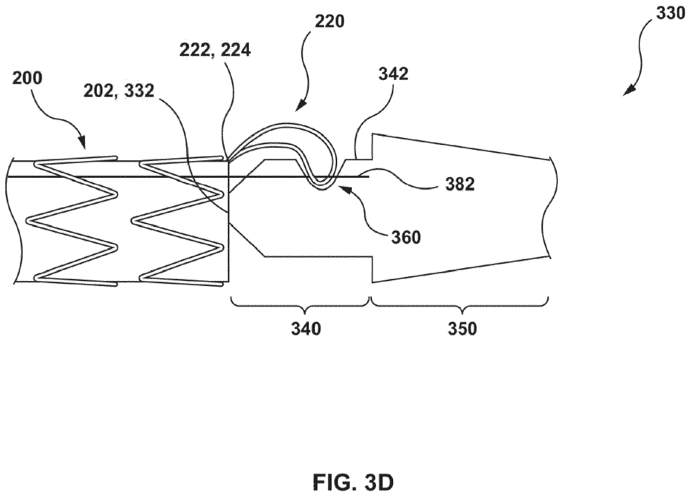

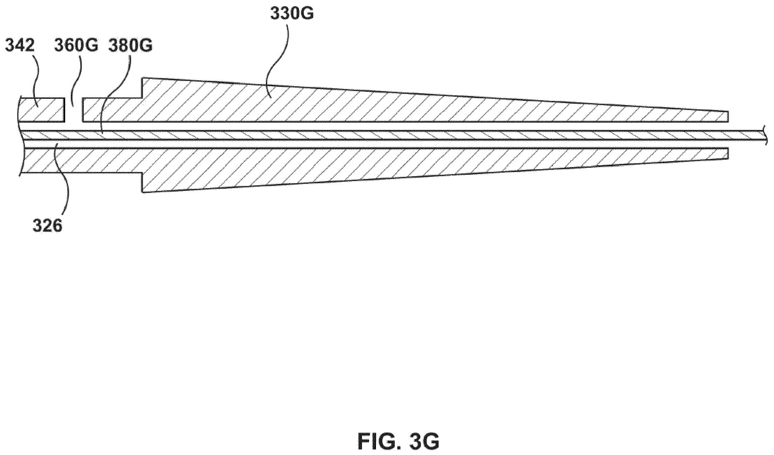

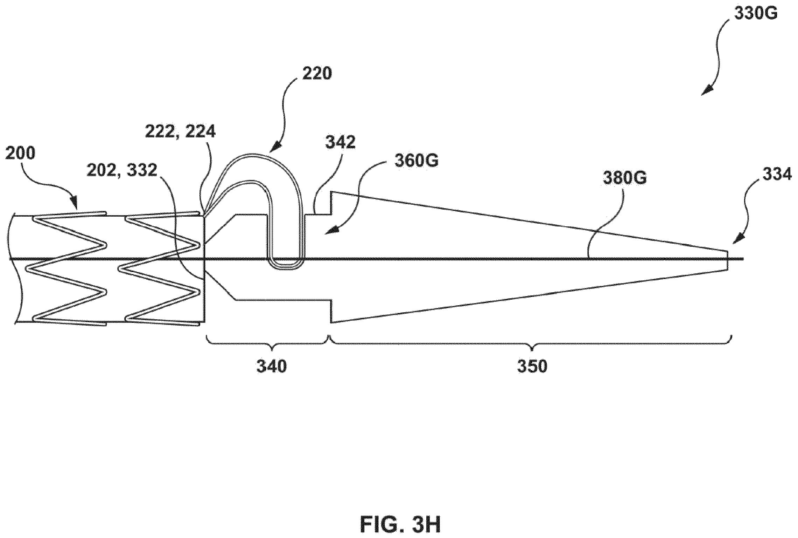

FIGS. 3-3D illustrate a stent-graft delivery catheter 300 that may be utilized to deliver the stent-graft 200 according to an embodiment hereof. It will be understood by one of ordinary skill in the art that the delivery catheter 300 is merely exemplary and may include additional components than those described herein. The delivery catheter 300 includes an elongate outer sheath 310, an elongate inner shaft 320 having a self-expanding stent-graft prosthesis, such as the stent-graft 200 shown and described above, mounted on a distal portion thereof, and a distal tip 330 coupled to the inner shaft 320. The outer sheath 310 is slidingly disposed over the inner shaft 320 to retain the stent-graft 200 in a radially constrained or compressed delivery configuration while the delivery catheter 300 is tracked through a patient's vasculature 100 to the deployment site. In FIG. 3, the outer sheath 310 is shown in a non-retracted, delivery configuration and extends over the stent-graft 200 to restrain the stent-graft 200 in the compressed delivery configuration.

The outer sheath 310 defines a central lumen 316 extending from a proximal end 312 to a distal end 314. The outer sheath 310 is moveable in an axial direction along and relative to the inner shaft 320 and extends to a proximal portion of the delivery catheter 300 where it may be controlled via an actuator, such as a handle 306. The handle 306 may be a push-pull actuator that is attached or connected to the proximal end 312 of the outer sheath 310. Alternatively, the actuator may be a rotatable knob (not shown) that is attached or connected to the proximal end 312 of the outer sheath 310 such that when the knob is rotated, the outer sheath 310 is retracted in a proximal direction to expand the stent-graft 200. The outer sheath 310 may include any suitable flexible polymeric material, including but not limited to polyethylene terephalate (PET), nylon, polyethylene, PEBAX, or combinations thereof.

FIG. 3A is a cross-sectional view of the delivery catheter 300 taken along line A-A of FIG. 3. In an assembled configuration, the inner shaft 320 is disposed within the central lumen 316 of the outer sheath 310, as shown in FIG. 3A. Prior to delivery of the stent-graft 200, the stent-graft 200 is radially compressed and is loaded onto an outer surface of the inner shaft 320. The outer sheath 310 is configured to slide over the inner shaft 320 and the stent-graft 200 disposed thereon such that the outer sheath 310 covers or houses the stent-graft 200 during tracking and delivery to a target site within the vasculature 100. When in use, the handle 306 and the proximal ends of the outer sheath and inner shaft 310, 320 of the delivery catheter 300 extend outside of the patient's vasculature 100.

The inner shaft 320 defines a central lumen 326A for receiving a guidewire 380 therethrough. The central lumen 326A extends from a proximal end 322 to a distal end 324 of the inner shaft 320. The inner shaft 320 also includes a longitudinally-extending lumen 328A for receiving a trigger wire 382 therethrough. The longitudinally-extending lumen 328A extends or is formed within a sidewall of the inner shaft 320, and extends from the proximal end 322 to the distal end 324 of the inner shaft 320. The inner shaft 320 may be constructed from any suitable flexible polymeric material, including but not limited to polyethylene terephalate (PET), nylon, polyethylene, PEBAX, or combinations thereof.

The delivery catheter 300 also includes the distal tip 330, which is attached to and distally extends from the distal end 322 of the inner shaft 320. FIG. 3B is a cross-sectional view taken along line B-B of FIG. 3, while FIG. 3C is a sectional view of the distal tip 330 taken along line C-C of FIG. 3. The distal tip 330 includes a proximal end 332, a distal end 334, and a central tip lumen 326B extending from the proximal end 332 to the distal end 334. The central tip lumen 326B is in fluid communication with the central lumen 326A of the inner shaft 320, which collectively form a guidewire lumen 326 of the delivery catheter 300. The distal tip 330 also includes a longitudinally-extending tip lumen 328B for receiving the trigger wire 382 therethrough. The longitudinally-extending tip lumen 328B extends or is formed within a sidewall of the distal tip 330. More particularly, the longitudinally-extending tip lumen 328B extends from the proximal end 332 of the distal tip 330 and terminates within the sidewall of the distal tip, as best shown in FIG. 3C. The longitudinally-extending tip lumen 328B is in fluid communication with the longitudinally-extending lumen 328A of the inner shaft 320, which collectively form a trigger wire lumen 328 of the delivery catheter 300.

The distal tip 330 includes a proximal portion 340 and a distal portion 350, as shown best in FIG. 3C. The proximal portion 340 of the distal tip 330 includes a substantially cylindrical-shaped sidewall 342. The distal portion 350 of the distal tip 330 is substantially conical-shaped. The distal portion 350 of the distal tip 330 tapers in a distal direction such that a first diameter of the proximal end is greater than a second diameter of the distal end of the distal portion 350 of the distal tip 330. The proximal portion 340 is sized and configured to be disposed within the central lumen 316 of the outer sheath 310 when the outer sheath 310 extends over the stent-graft 200, with the distal end 314 of the outer sheath 310 abutting against and substantially flush with the proximal end of the distal portion 350 of the distal tip 330. The distal tip 330 may be formed from any suitable material, including but not limited to, PEBAX, urethane, silicone, other flexible polymers, and any other materials known to those skilled in the art.

The distal tip 330 includes a slot 360 disposed in the sidewall 342 of the distal tip 330. In an embodiment, the slot 360 is disposed adjacent to the proximal end of the distal tip 330. The slot 360 of the distal tip 330 extends through a portion of the sidewall 342 such that the slot 360 is in fluid communication with the longitudinally-extending tip lumen 328B of the distal tip 330, and thus the trigger wire lumen 328 of the delivery catheter 300. The slot 360 exposes a portion of the trigger wire 382 that is disposed within the trigger wire lumen 328 of the delivery catheter 300. The combination of the slot 360 of the distal tip 330 and the trigger wire 382 of the delivery catheter 300 form a retention feature of the delivery catheter 300.

The retention feature of the delivery catheter 300 is configured to interact with the suture loop 220 of the stent-graft 200. More particularly, when the stent-graft 200 is loaded into the delivery catheter 300, the proximal end 202 of the stent-graft 200 is disposed adjacent to the proximal end of the distal tip 330 and the suture loop 220 extends through the slot 360 and into the trigger wire lumen 328. Prior to delivery, a distal end of the trigger wire 382 of the delivery catheter 300 is advanced or threaded distally through the trigger wire lumen 328 until it reaches the distal tip 330. As the distal end of the trigger wire 382 is advanced through the distal tip 330, the distal end of the trigger wire 382 is also advanced through the suture loop 220 of the stent-graft 200 that is disposed within the trigger wire lumen 328. Stated another way, after the suture loop 220 is inserted through the slot 360, the distal end of the trigger wire 382 is advanced through the suture loop 220 of the stent-graft 200, as shown in FIG. 3D. The cross-section of the slot 360 within the sidewall 342 of the distal tip 330 can be any suitable shape, such as but not limited to circular or oval. In addition, the size or dimension of the cross-section of the slot 360 may remain constant along the depth, as shown in FIG. 3C, or the size or dimension of the cross-section of the slot 360 may taper or vary along the depth, as shown in FIG. 3D. The cross-section of the slot 360 preferably allows the suture loop 220 to easily exit the slot 360 of the distal tip 330 when the trigger wire 382 is retracted. The tapered or sloped edges of the slot 360 shown in FIG. 3D may facilitate release or exiting of the trigger wire 382 from the slot 360. In some embodiments, the slot 360 of the distal tip 330 is formed by removing the least amount of material from the distal tip 330 to maintain the strength of the distal tip 330 structure. The distal end of the trigger wire 382 continues to advance distally until it reaches a distal end of the trigger wire lumen 328. The trigger wire 382 of the delivery catheter 300 retains the suture loop 220 of the stent-graft 200 and effectively couples the proximal end 202 of the stent-graft 200 to the delivery catheter 300 until the trigger wire 382 is retracted, which will be described in further detail below.

As best shown on FIGS. 3E and 3F, the length of the suture loop 220 is configured to engage the trigger wire 382 via the slot 360 when the proximal end 202 of the stent-graft 200 is fully radially expanded or deployed. The proximal end 202 of the stent-graft 200 is permitted to radially expand or deploy, while the suture loop 220 remains engaged with the trigger wire 382 via the slot 360. Thus, the retention feature of the delivery catheter 300 maintains or retains the position of the deployed proximal end 202 of the stent-graft 200 and prevents distal migration during subsequent steps of the procedure.

Notably, by including exactly one suture loop 220 on the proximal end 202 of the stent-graft 200, the amount or area of the proximal body lumen 211 at the proximal end 202 of the stent-graft 200 that is constrained by the suture loop 220 is minimized, as best shown in FIG. 3F. As additional procedures are conducted after the deployment and expansion of the stent-graft 200 within the vasculature 100, only a minimal area of the proximal end 202 of the stent-graft 200 is constrained by the suture loop 220. Using more than one suture loop 220 to retain the proximal end 202 of the stent-graft 200 to the distal tip 330 of the delivery catheter 300 would undesirably reduce the proximal body lumen 211 of the stent-graft 200, making it harder to maneuver subsequent delivery systems through the proximal body lumen 211 of the stent-graft 200. In an embodiment, the suture loop 220 is configured to constrain less than 20% of the perimeter of the stent-graft 200 when the stent-graft 200 is in the radially expanded configuration.

In another embodiment hereof, which is depicted in FIGS. 3G and 3H, the trigger wire and the trigger lumen of the delivery system may be omitted and the guidewire can function as the trigger wire. More particularly, FIG. 3G illustrates a sectional view of a distal tip 330G according to another embodiment hereof. The distal tip 330G is similar to the distal tip 330, except that the longitudinally-extending tip lumen 328B is omitted and a slot 360G has a different configuration than the slot 360. More particularly, the slot 360G of the distal tip 330G extends through the entire sidewall 342 such that the slot 360G is in fluid communication with the central tip lumen 326B of the distal tip 330G, and thus a guidewire 380G of the delivery system. The slot 360G exposes a portion of the guidewire 380G that is disposed within the guidewire lumen 326 of the delivery catheter 300. The combination of the slot 360G of the distal tip 330G and the guidewire 380G of the delivery system form a retention feature of the delivery system, which is configured to interact with the suture loop 220 of the stent-graft 200. More particularly, as shown on FIG. 3H, when the stent-graft 200 is loaded into the delivery catheter 300, the proximal end 202 of the stent-graft 200 is disposed adjacent to the proximal end of the distal tip 330G and the suture loop 220 extends through the slot 360G, into the guidewire lumen 326 of the delivery system. After the suture loop 220 is inserted through the slot 360G, the guidewire 380G of the delivery system retains the suture loop 220 of the stent-graft 200 and effectively couples the proximal end 202 of the stent-graft 200 to the delivery system until the guidewire 380G is retracted.

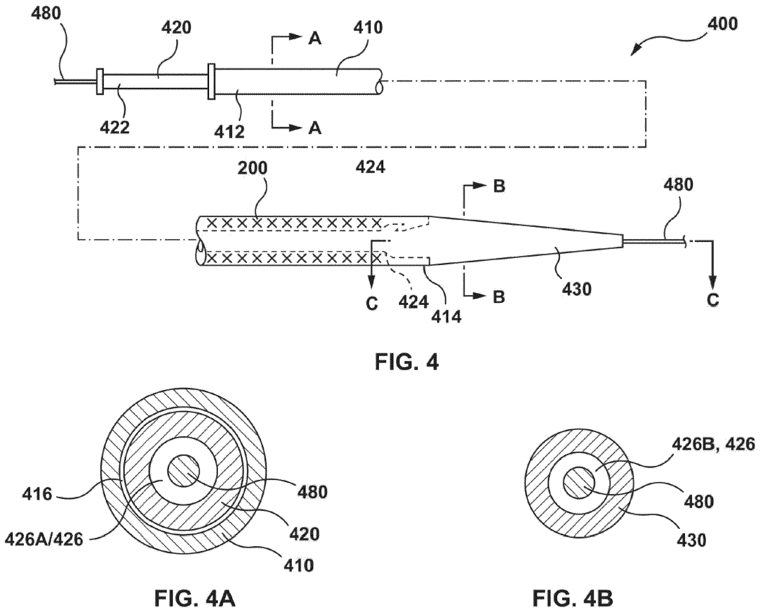

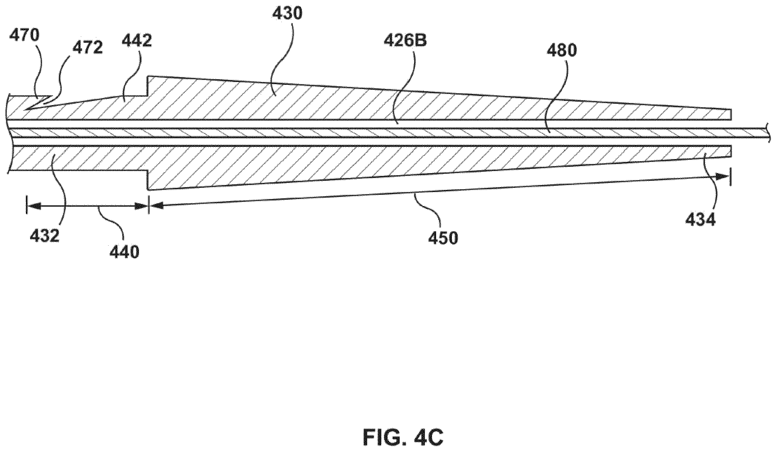

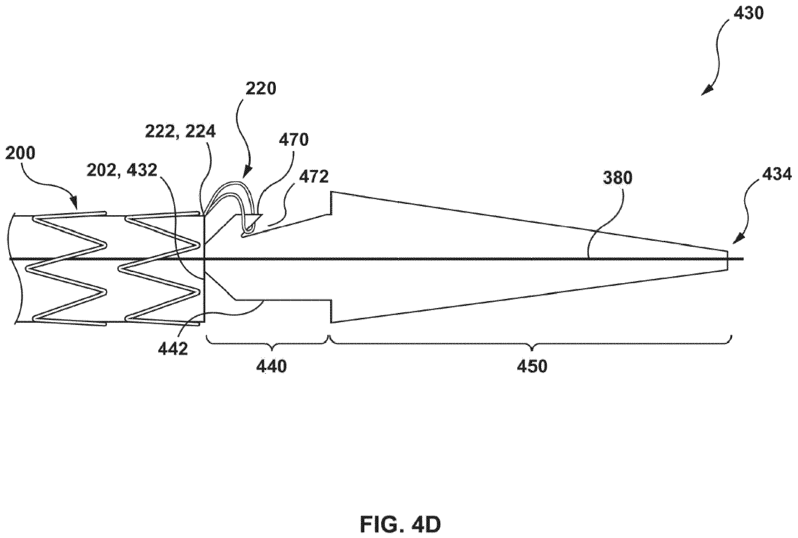

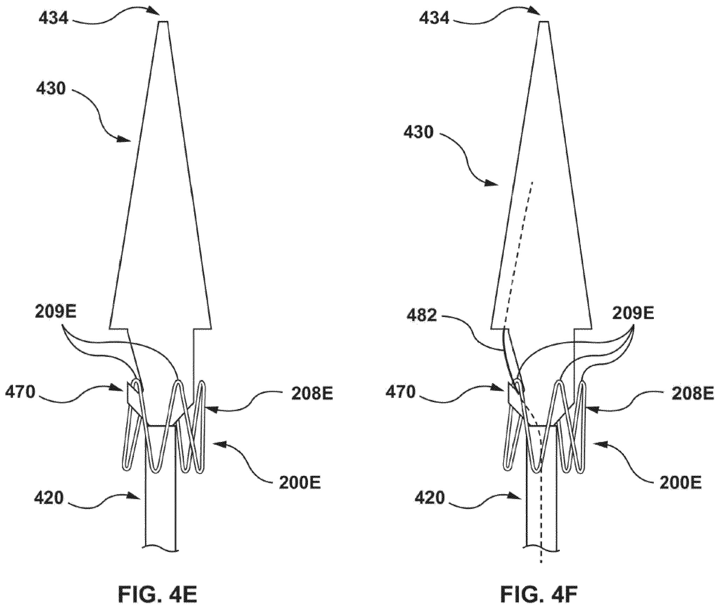

FIGS. 4-4D show another embodiment of a retention feature according to another embodiment hereof. The delivery catheter 400 is similar to the delivery catheter 300, except for the differences described herein. The delivery catheter 400 includes an elongate outer sheath 410, an elongate inner shaft 420 having a self-expanding stent-graft prosthesis, such as the stent-graft 200 shown and described above, mounted on a distal portion thereof, and a distal tip 430 coupled to the inner shaft 420. The outer sheath 410 is slidingly disposed over the inner shaft 420 to retain the stent-graft 200 in a radially constrained or compressed delivery configuration while the delivery catheter 400 is tracked through a patient's vasculature 100 to the deployment site. In FIG. 4, the outer sheath 410 is shown in a non-retracted, delivery configuration and extends over the stent-graft 200 to restrain the stent-graft 200 in the compressed delivery configuration.

FIG. 4A is a cross-sectional view of the delivery catheter 400 taken along line A-A of FIG. 4. The outer sheath 410 defines a central lumen 416 extending from a proximal end 412 to a distal end 414. The inner shaft 420 defines a central lumen 426A for receiving a guidewire 480 therethrough. The central lumen 426A extends from a proximal end 422 to a distal end 424 of the inner shaft 420. In an assembled configuration, the inner shaft 420 is disposed within the central lumen 416 of the outer sheath 410, as shown in FIG. 4A.

The delivery catheter 400 also includes the distal tip 430, which is attached to and distally extends from the distal end 424 of the inner shaft 420. FIG. 4B is a cross-sectional view taken along line B-B of FIG. 4, while FIG. 4C is a sectional view of the distal tip 430 taken along line C-C of FIG. 4. The distal tip 430 includes a proximal end 432, a distal end 434, and a central tip lumen 426B extending from the proximal end 432 to the distal end 434. The central tip lumen 426B is in fluid communication with the central lumen 426A of the inner shaft 420, which collectively form a guidewire lumen 426 of the delivery catheter 400. The distal tip 430 includes a proximal portion 440 and a distal portion 450, as shown best in FIG. 4C. The proximal portion 440 of the distal tip 430 includes a substantially cylindrical-shaped sidewall 442. The distal portion 450 of the distal tip 430 is substantially conical-shaped. The distal portion 450 of the distal tip 430 tapers in a distal direction such that a first diameter of the proximal end is greater than a second diameter of the distal end of the distal portion 450 of the distal tip 430. The proximal portion 440 is sized and configured to be disposed within the central lumen 416 of the outer sheath 10 when the outer sheath 410 extends over the stent-graft 200, with the distal end 414 of the outer sheath 410 abutting against and substantially flush with the proximal end of the distal portion 450 of the distal tip 430.

The distal tip 430 includes a distal-facing notch 470, also referred to as a protrusion or hook, disposed or formed on the sidewall 442 of the distal tip 430. The distal-facing notch 470 is disposed adjacent to the proximal end of the distal tip 430. The distal-facing notch 470 is formed by a slit 472 that extends through a portion of the sidewall 442 of the distal tip 430, resulting in a portion of the sidewall 442 that protrudes radially outward from the sidewall 442 and extends in a distal direction towards the distal end of the distal tip 430, as can be best seen in FIG. 4C. The protrusion that results from the slit 472 in the sidewall of the distal tip 430 is the distal-facing notch 470, i.e., a protrusion that extends towards the distal end of the distal tip 430. A distal surface of the distal-facing notch 470 forms an acute angle relative to a longitudinal axis of the delivery catheter 400. In an embodiment, the acute angle may be between 30 degrees and 60 degrees. In another embodiment, the acute angle may be 45 degrees, with a tolerance of at least 5 degrees.

The distal-facing notch 470 of the distal tip 430 forms a retention feature of the delivery catheter 400, as best shown in FIG. 4D. The retention feature of the delivery catheter 400 is configured to interact with the suture loop 220 of the stent-graft 200. More particularly, prior to delivery, the suture loop 220 of the stent-graft 200 engages or hooks onto the distal-facing notch 470 of the distal tip 430 such that the proximal end 202 of the stent-graft 200 is effectively coupled to the delivery catheter 400 until the inner shaft 420 of the delivery catheter 400 is retracted. The length of the suture loop 220 is configured to engage the distal-facing notch 470 when the proximal end 202 of the stent-graft 200 is fully radially expanded or deployed. The proximal end 202 of the stent-graft 200 is permitted to radially expand or deploy, while the suture loop 220 remains engaged with the distal-facing notch 470. Thus, the retention feature of the delivery catheter 400 maintains or retains the position of the deployed proximal end 202 of the stent-graft 200 and prevents distal migration during subsequent steps of the procedure.

Due to the orientation of the distal-facing notch 470, the suture loop 220 can be released from the distal-facing notch 470 of the distal tip 430 when the inner shaft 420 and the distal tip 430 of the delivery catheter 400 are retracted and withdrawn from the vasculature 100. If the slit 472 resulted in the protrusion 470 facing proximally, i.e. toward the proximal end 332 of the distal tip 430, this release method would not be possible. Thus, the distal-facing notch 470 of the distal tip 430 allows the suture loop 220 of the stent-graft 200 to disengage when the delivery catheter 400 is being retracted from the vasculature 100. No additional tools, delivery system features, or methods are needed to disengage the suture loop 220 from the distal-facing notch 470.

In another embodiment depicted in FIG. 4E, as an alternative to the suture loop 220, a crown 209E of the stent-graft 200E may be used to engage a retention feature of the delivery system. More particularly, an exposed or uncovered crown 209E of a stent-graft 200E may be hooked and secured onto the distal-facing notch 470 of the distal tip 430, as shown in FIG. 4E. In such an embodiment, the stent-graft 200E includes an open web configuration, such that the graft material does not cover the endmost crowns 209E of the proximal-most stent ring 208E. Stated another way, the endmost crowns 209E of the proximal-most stent ring 208E extend past the proximal end of the graft material such that the endmost crowns 209E of the proximal-most stent ring 208E are exposed and may be hooked and secured on the distal-facing notch 470 of the distal tip 430. In such embodiments, the delivery catheter 400 does not require a trigger wire, but a trigger wire 482 may be included for redundancy, as shown in the embodiment of FIG. 4F.

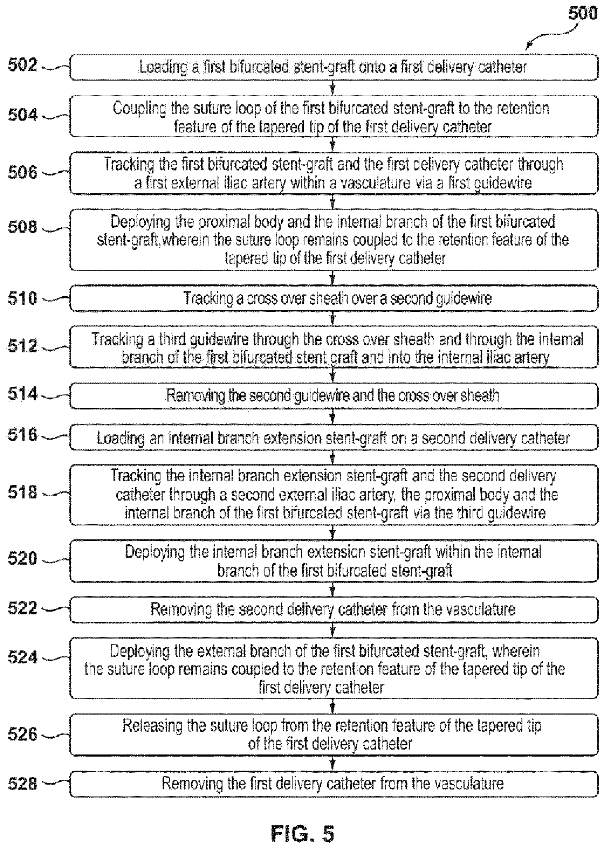

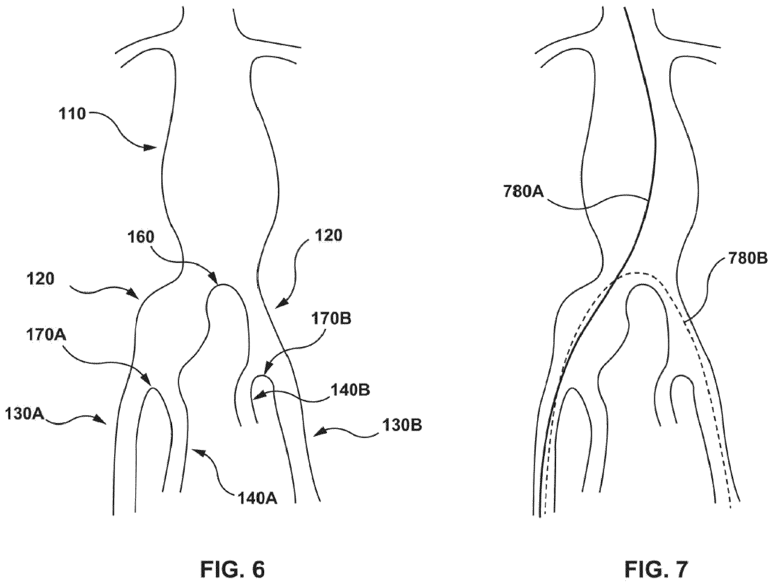

FIG. 5 is a block diagram of a method 500 for delivering a first stent-graft 200 and one or more additional stent-grafts therewithin using the retention systems described above. The method described herein is directed to delivering a first bifurcated stent-graft within a common iliac artery, as shown in FIG. 6, and deploying additional stent-grafts throughout the patient's vasculature that may overlap with the first bifurcated stent-graft. However, this is not meant to be limiting, as the retention systems described and disclosed herein may be used for deploying various types of stents at any location within the body to reduce stent compression and migration during delivery and deployment.

In a first step 502 of the method 500, a first bifurcated stent-graft 200, such as the stent-graft shown in FIG. 2, is loaded onto a first delivery system or catheter 300, 400 such as the delivery catheter described above with respect to FIGS. 3A-4F. As stated previously, the first bifurcated stent-graft 200 is radially compressed and loaded onto an outer surface of the inner shaft 320, 420 of the delivery catheter 300, 400. The outer sheath 310, 410 is then slid over the inner shaft 320, 420 and the radially-compressed first bifurcated stent-graft 200 such that the inner shaft 320, 420 and the loaded first bifurcated stent-graft 200 are disposed within the central lumen 316, 416. The first bifurcated stent-graft 200 is loaded onto a distal portion of the inner shaft 320, 420 such that the proximal end 202 of the stent-graft 200 is disposed adjacent to the proximal end 332, 432 of the distal tip 330, 430 of the delivery catheter 300, 400.

In a second step 504 of the method 500, the suture loop 220 of the first bifurcated stent-graft 200, coupled to the proximal end 202 of the first bifurcated stent-graft 200, is coupled to the retention feature of the distal tip 330, 330G, 430. In a first embodiment, a trigger wire 382 extends through the suture loop 220 of the first bifurcated stent-graft 200 at the slot 360 formed in the sidewall 342 of the distal tip 330, as shown and described with respect to the retention feature in FIGS. 3A-3D. In an alternate first embodiment, a guidewire 380G extends through the suture loop 220 of the first bifurcated stent-graft 200 at the slot 360G formed in the sidewall 342 of the distal tip 330G, as shown and described with respect to the alternate retention feature in FIGS. 3G-3H. In a second embodiment, the suture loop 220 is hooked and secured onto the distal-facing notch 470 of the distal tip 430, as shown and described with respect to the retention feature in FIGS. 4C-4D. In a third embodiment, a crown 209E of a proximal-most stent ring 208E is secured onto a notch (e.g., distal-facing notch 470) and a trigger wire 482 is optionally included through the crown 209E. Depending on the type of loading method used, step 504 may take place prior to step 502.

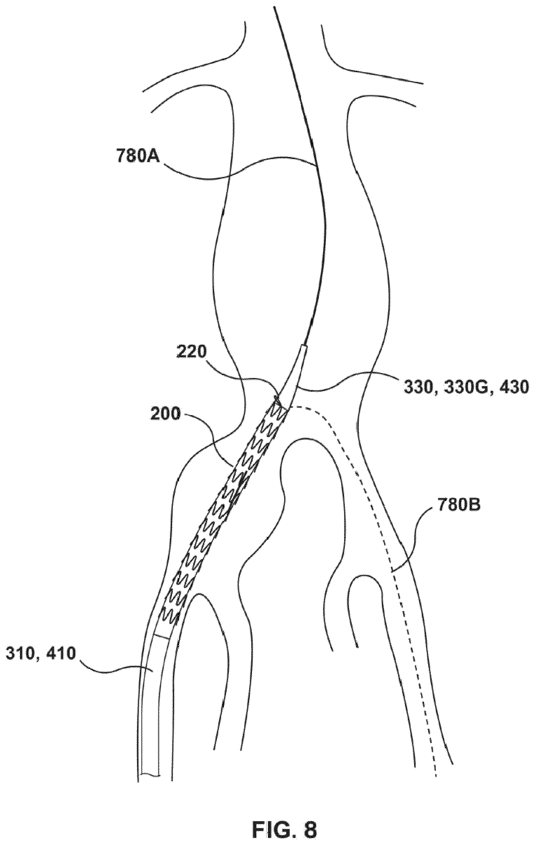

In a third step 506 of the method 500, the first delivery catheter 300, 400 and the first bifurcated stent-graft 200 loaded thereon, are tracked through a first (in FIGS. 6-16, left) external iliac artery 130A within the vasculature 100 of the patient via a first guidewire 780A. To do this, a distal end of the first guidewire 780A gains access to the first external iliac artery 130A via a needle and is tracked towards the aorta 110 of the patient, as can be seen in FIG. 7. In this step, a second guidewire 780B gains access to a second (in FIGS. 6-16, right) external iliac artery 130B via a needle and is tracked up and over the first bifurcation 160 of the vasculature 100 and down into the first external iliac artery 130A to prepare for a subsequent step. The first delivery catheter 300, 400 is then loaded onto the first guidewire 780A and is tracked over the first guidewire 780A until the distal tip 330, 330G, 430 of the delivery catheter 300, 400 reaches the aorta 110 of the patient, as shown in FIG. 8. The first delivery catheter 300, 400 may optionally also be tracked over the second guidewire 780B (as shown in FIG. 8), allowing the bifurcated stent-graft 200 to have the second guidewire 780B pre-cannulated through the internal branch 212 thereof. The second guidewire 780B may extend through a notch or channel on the outer periphery of the tip 330, 330G, 430 of the delivery catheter 300, 400.

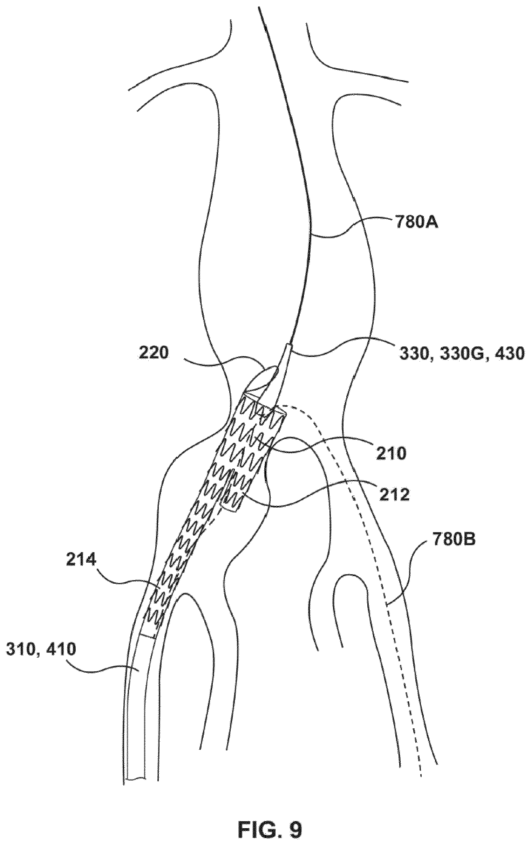

In a fourth step 508 of the method 500, the proximal body 210 and the internal branch 212 of the first bifurcated stent-graft 200 are deployed. To do this, the outer sheath 310, 410 of the delivery catheter 300, 400 is partially retracted proximally such that the proximal body 210 and the internal branch 212 of the first bifurcated stent-graft 200 are uncovered and are able to fully expand radially, as shown in FIG. 9. During this step, the suture loop 220 coupled to the proximal end 202 of the first bifurcated stent-graft 200 remains coupled and secured to the retention feature of the delivery catheter 300, 400 (e.g., the slot 360, 360G of the distal tip 330, 330G or the distal-facing notch 470 of the distal tip 430). At the end of this step, the external branch 214 of the first bifurcated stent-graft 200 is still enclosed within the outer sheath 310, 410 of the delivery catheter 300, 400 as can be seen in FIG. 9. In addition, at the end of this step, the second guidewire 780B extends through the proximal body 210 and the internal branch 212 of the first bifurcated stent-graft 200, as shown in FIG. 9.

In a fifth step 510 of the method 500, a cross over sheath 1000 is loaded on the second guidewire 780B. The cross over sheath 1000 is tracked over the second guidewire 780B and through the proximal end 202, the proximal body 210 and the internal branch 212 of the first bifurcated stent-graft 200, as shown in FIG. 10. In an embodiment, the suture loop 220 is oriented on the proximal end 202 of the first bifurcated stent-graft 200, on a side opposite of where the cross over sheath 1000 enters the proximal end 202 of the first bifurcated stent-graft 200 such that the suture loop 220 does not disrupt the path of the cross over sheath 1000, as shown in FIG. 10. In this preferred circumferential position of suture loop 220, the first guidewire 780A pulls the suture loop 220, and the external branch 214 of the first bifurcated stent-graft 200, away from the second guidewire 780B which is routed through the internal branch 212 of the first bifurcated stent-graft 200. This interaction favors or stabilizes the preferred rotational alignment of the first bifurcated stent-graft 200 within the vessel as the cross over sheath 1000 is advanced through the internal branch 212.

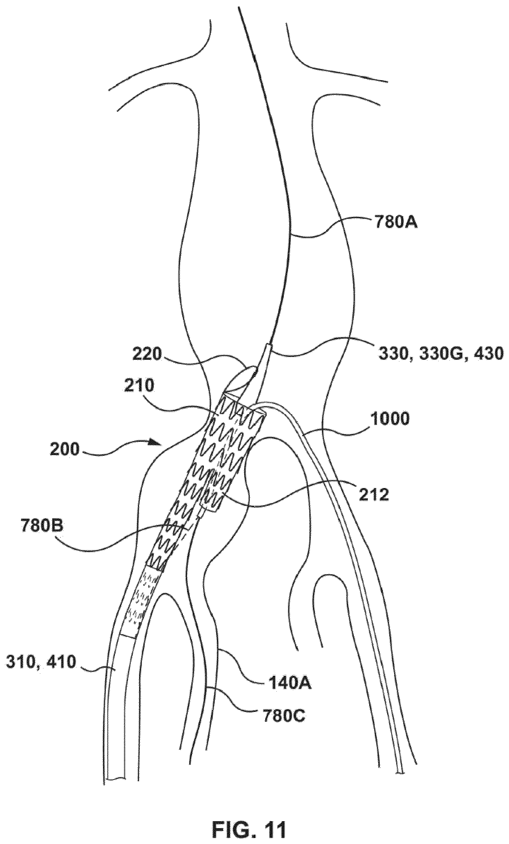

In a sixth step 512 of the method 500, a third guidewire 780C is tracked through a lumen of the cross over sheath 1000 such that the third guidewire 780C extends through the proximal body 210 and the internal branch 212 of the first bifurcated stent-graft 200 and a distal end of the third guidewire 780C is disposed within a first or left internal iliac artery 140A of the patient's vasculature 100, as shown in FIG. 11. The cross over sheath 1000 permits positioning and delivery of the stiffer third guidewire 780C into the first internal iliac artery 140A.

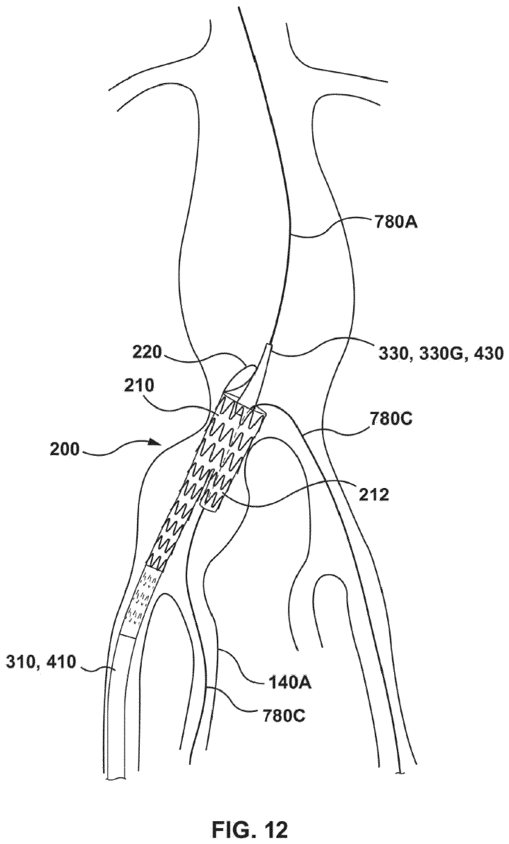

In a seventh step 514 of the method 500, the cross over sheath 1000 and the second guidewire 780B are removed from the patient's vasculature 100, leaving only the third guidewire 780C in place as shown in FIG. 12. To do this, the cross over sheath 1000 and the second guidewire 780B are proximally retracted until they are both removed from the vasculature 100 of the patient. In some embodiments, the cross over sheath 1000 and the second guidewire 780B remain within the vasculature 100 of the patient for additional steps of the method of use. For example, it may be desirable to leave the second guidewire 780B in place until a stent-graft extension is deployed within the first internal iliac artery 140A in case access to the first internal iliac artery 140A via the third guidewire 780C is inadvertently lost during the procedure. As another example, it may be desirable to leave the cross over sheath 1000 in place until a stent-graft extension is deployed within the first internal iliac artery 140A so that the stent-graft extension may be tracked or advanced through the cross-over sheath 1000. The cross over sheath 1000 may be only slightly retracted, rather than retracted and withdrawn, such that the stent-graft extension is permitted to expand distal to or clear of the cross over sheath 1000.

In an eighth step 516 of the method 500, an internal branch extension stent-graft 1200 is loaded on a second delivery catheter 1300. Similar to the first-bifurcated stent-graft 200, the internal branch extension stent-graft 1200 is radially compressed and loaded onto an inner shaft 1320 of the second delivery catheter 1300 and an outer sheath 1310 of the second delivery catheter 1300 slides over the inner shaft 1320 and the internal branch extension stent-graft 1200 such that the inner shaft 1320 and the internal branch extension stent-graft 1200 are disposed within an outer shaft lumen 916 of the outer sheath 1310. The internal branch extension stent-graft 1200 may be a balloon-expandable stent-graft or a self-expanding stent-graft. If it is balloon-expandable, the second delivery catheter 1300 may include a balloon and corresponding inflation lumen(s) and components as known to those of ordinary skill in the art.

In a ninth step 518 of the method 500, the second delivery catheter 1300 and the internal branch extension stent-graft 1200 disposed thereon are tracked over the third guidewire 780C and are advanced through the proximal body lumen 211 of the first bifurcated stent-graft 200 at the proximal end 202. During this step, the suture loop 220 coupled to the proximal end 202 of the first bifurcated stent-graft 200 is still coupled and secured to the retention feature on the distal tip 330, 330G, 430 of the first delivery catheter 300, 400. The second delivery catheter 1300 is then advanced into the internal branch lumen 213 of the first bifurcated stent-graft 200 and into the first internal iliac artery 140A.

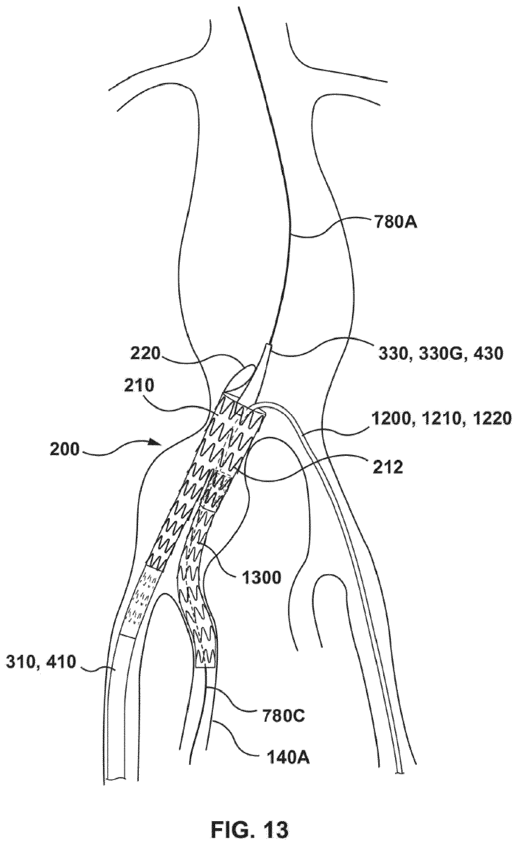

In a tenth step 520 of the method 500, the internal branch extension stent-graft 1200 is deployed within the first internal iliac artery 140A of the vasculature 100, as shown in FIG. 12. To do this, the outer sheath 1310 of the second delivery catheter 1300 is proximally retracted such that the internal branch extension stent-graft 1200 is able to expand radially. As shown in FIG. 13, a proximal portion of the internal branch extension stent-graft 1200 overlaps and expands within the internal branch 212 of the first bifurcated stent-graft 200. If the internal branch extension stent-graft 1200 is a balloon-expandable stent-graft, then an inflation step is performed to expand the stent-graft and an anchor is disposed within the internal branch 212 of the first bifurcated stent-graft 200. Even if the internal branch extension stent-graft 1200 is self-expanding, an accessory balloon may be expanded at the overlap region with the internal branch 212 to improve the seal between the two components.

In an eleventh step 522 of the method 500, the second delivery catheter 1300 and the third guidewire 780C are proximally retracted and removed from the vasculature 100 of the patient. In some embodiments, as described above, the cross over sheath 1000 and the second guidewire 780B remain within the vasculature 100 of the patient until this step is completed. Then, the cross over sheath 1000 and the second guidewire 780B are proximally retracted until they are both removed from the vasculature 100 of the patient.

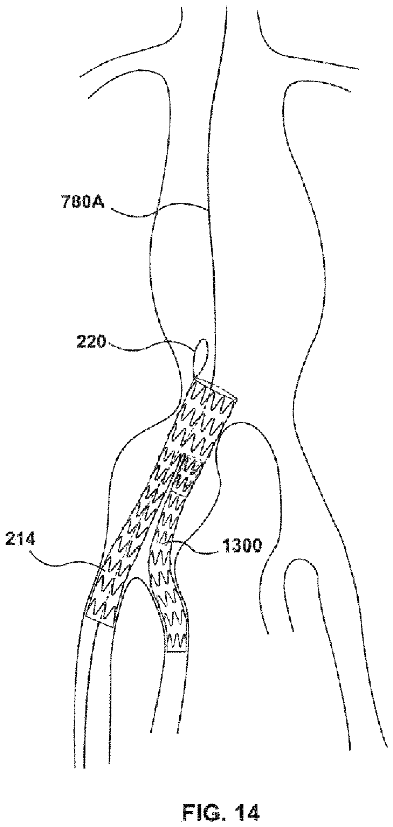

In a twelfth step 524 of the method 500, the external branch 214 of the first bifurcated stent-graft 200 is deployed, as shown in FIG. 14. To do this, the outer sheath 310, 410 of the first delivery catheter 300, 400 is retracted fully such that the external branch 214 of the first bifurcated stent-graft 200 is uncovered and is able to fully expand. During this step, the suture loop 220 coupled to the proximal end 202 of the first bifurcated stent-graft 200 remains coupled and secured to the retention feature on the distal tip 330, 330G, 430 of the first delivery catheter 300, 400. In some embodiments, as described above, the cross over sheath 1000 and the second guidewire 780B remain within the vasculature 100 of the patient until this step is completed. Then, the cross over sheath 1000 and the second guidewire 780B are proximally retracted until they are both removed from the vasculature 100 of the patient.

In a thirteenth step 526 of the method 500, the suture loop 220 of the first bifurcated stent-graft 200 is released from the retention feature on the distal tip 330, 330G, 430 of the delivery catheter 300, 400. In the first embodiment, the trigger wire 382 extends through the suture loop 220 at the slot 360 that extends through the sidewall 342 of the distal tip 330. To release the suture loop 220, the trigger wire 382 is retracted proximally such that the trigger wire 382 is removed from the first delivery catheter 300 and thus, removed from the suture loop 220 of the first bifurcated stent-graft 200. Once the trigger wire 382 is removed, the first bifurcated stent-graft 200 is no longer secured or coupled to the first delivery catheter 300.

In the alternate first embodiment, the guidewire 380G extends through the suture loop 220 at the slot 360G that extends through the sidewall 342 of the distal tip 330G. To release the suture loop 220, the guidewire 380G is retracted proximally such that the guidewire 380G is removed from the distal tip 330G of the delivery catheter 300 and thus, removed from the suture loop 220 of the first bifurcated stent-graft 200. Once the guidewire 380G is removed from the suture loop 220, the first bifurcated stent-graft 200 is no longer secured or coupled to the first delivery catheter 300.

In the second embodiment, the suture loop 220 is secured to the distal-facing notch 470 on the distal tip 430 of the first delivery catheter 400. To detach the suture loop 220 of the first bifurcated stent-graft 200 from the distal-facing notch 470, the entirety of the first delivery catheter 400, including the distal tip 430 and the inner shaft 420, is retracted proximally such that the suture loop 220 can disengage from the distal-facing notch 470 and is no longer disposed within the slit 472 of the distal-facing notch 470. Once the first delivery catheter 400 is retracted proximally and the suture loop 220 detaches from the distal-facing notch 470, the first bifurcated stent-graft 200 is no longer secured or coupled to the first delivery catheter 400. Although the suture loop 220 shown and described in the method above, one of ordinary skill in the art would understand that the suture loop 220A shown and described with reference to FIG. 2A can be used in the method described above as well.

In a fourteenth step 528 of the method, the first delivery catheter 300, 400 is removed from the vasculature 100 of the patient. To do this, the first delivery catheter 300, 400 is retracted proximally such that it exits the proximal body lumen 211 and the external branch lumen 215 of the first bifurcated stent-graft 200 and exits the vasculature 100 of the patient, as shown in FIG. 14.

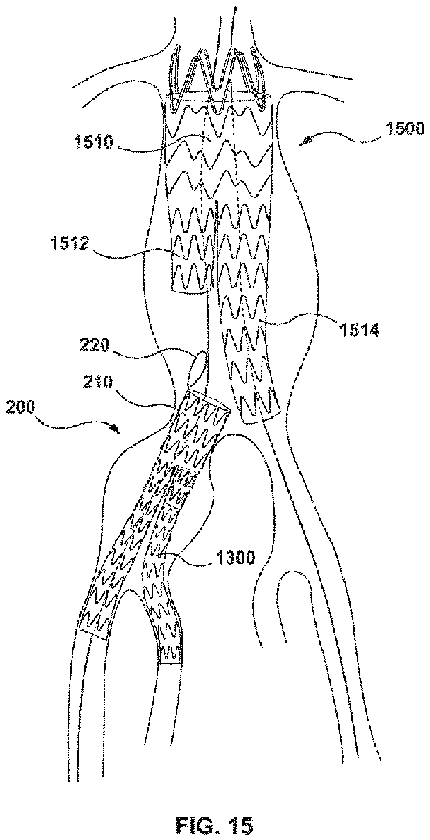

The method can further comprise deploying a second bifurcated stent-graft 1400 within the aorta 110 of the vasculature 100, as shown in FIG. 15. The second bifurcated stent-graft 1400 includes a proximal body 1410, a first or left branch 1412 and a second or right branch 1414 extending distally from the proximal body 1410 of the second bifurcated stent-graft 1400. The second bifurcated stent-graft 1400 can be tracked and delivered to the aorta 110 by a subsequent delivery system.

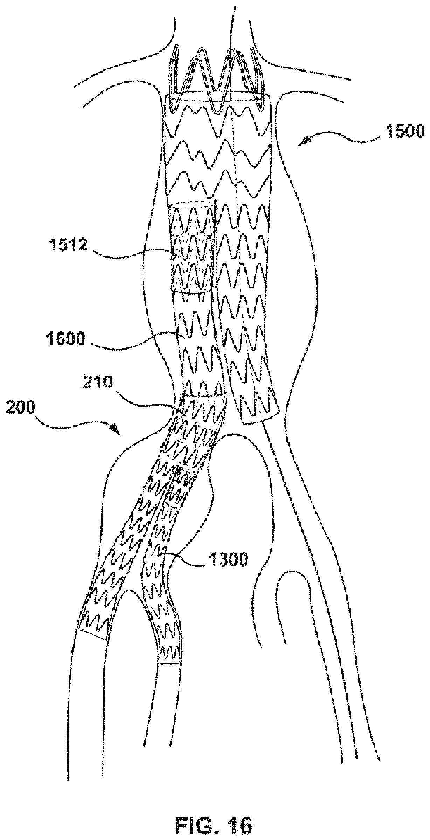

In addition, the method can further comprise deploying a first branch extension stent-graft 1500 that extends from the first branch 1412 of the second bifurcated stent-graft 1400 to the proximal body 210 of the first bifurcated stent-graft 200, as shown in FIG. 16. As can be seen, a distal end of the first branch extension stent-graft 1500 overlaps with the proximal body 210 of the first bifurcated stent-graft 200 and a proximal end of the first branch extension stent-graft 1500 overlaps with the left branch 1412 of the second bifurcated stent-graft 1400. The first branch extension stent-graft 1500 can be tracked and delivered to the aorta 110 by a subsequent delivery system.

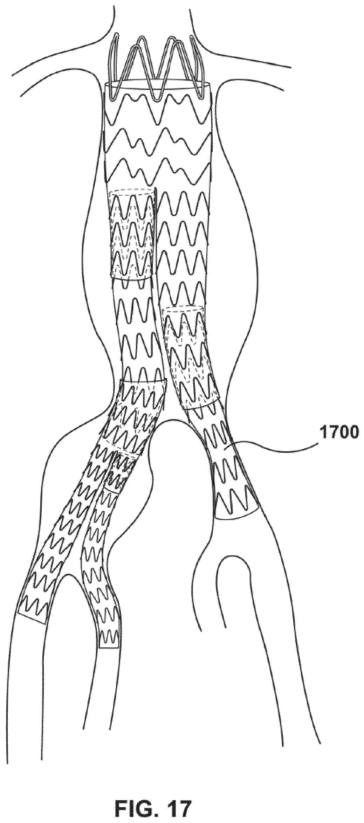

Lastly, the method can further comprise deploying a second branch extension stent-graft 1600 that extends from the second branch 1414 of the second bifurcated stent-graft 1400 to the second external iliac artery 130B of the vasculature 100, as shown in FIG. 17. As can be seen, a proximal end of the second branch extension stent-graft 1600 overlaps with the right branch 1414 of the second bifurcated stent-graft 1400. The second branch extension stent-graft 1500 can be tracked and delivered to the aorta 110 by a subsequent delivery system.

It should be understood that the retention system shown and described herein can be used with a bifurcated stent-graft. However, this is not meant to be limiting, as one of ordinary skill in the art would understand that the retention system shown and described herein can be used in conjunction with any stent-graft prosthesis. It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components.

Further disclosed herein is the subject-matter of the following clauses:
Clause 1. A retention system comprising:
   a stent-graft including:
      a proximal body having a proximal-most stent ring;
      a graft material; and
      a loop attached to a proximal end of the stent-graft, wherein the stent-graft has a radially compressed configuration for delivery within a vasculature and a radially expanded configuration for deployment, and
   a delivery catheter including:
      an inner shaft having a shaft lumen extending from a distal end to a proximal end of the inner shaft, wherein the stent-graft is disposed over a distal portion of the inner shaft in the radially compressed configuration;
      a distal tip coupled to the distal end of the inner shaft, the distal tip including a tip lumen and a slot extending through a sidewall of the distal tip, wherein the tip lumen is fluidly connected to the shaft lumen to form a trigger wire lumen of the delivery catheter; and
      a trigger wire extending through the trigger wire of the delivery catheter, wherein the slot of the distal tip exposes a portion of the trigger wire within the tip lumen,
   wherein the trigger wire extends through the loop of the stent-graft at the slot of the distal tip of the delivery catheter and is configured to retain the proximal end of the stent-graft in the radially expanded configuration via the loop to prevent distal migration and compression of the stent-graft, and
   wherein proximal retraction of the trigger wire releases the loop of the stent-graft such that the stent-graft is no longer coupled to the delivery catheter.
Clause 2. The retention system of claim 1, wherein the stent-graft is a bifurcated stent-graft, the bifurcated stent-graft having an internal branch and an external branch that extend distally from the proximal body.
Clause 3. The retention system of claim 1, wherein the loop is attached to the proximal-most stent ring at the proximal end of the stent-graft.
Clause 4. The retention system of claim 1, wherein the loop is attached to the graft material at the proximal end of the stent-graft.
Clause 5. The retention system of claim 1, wherein the trigger wire is a guidewire for the delivery catheter.
Clause 6. The retention system of claim 1, wherein the loop is a single loop such that the proximal end of the stent-graft includes exactly one loop.
Clause 7. The retention system of claim 1, wherein the loop has a length of about 3-20 mm.
Clause 8. The retention system of claim 1, wherein the loop is configured to constrain less than 20% of a perimeter of the stent-graft when the stent-graft is in the radially expanded configuration.
Clause 9. The retention system of claim 1, wherein the proximal-most stent ring of the stent-graft includes a plurality of proximal-most crowns.
Clause 10. The retention system of claim 9, wherein the loop is coupled to a single proximal-most crown of the proximal-most stent ring or is coupled to exactly two proximal-most crowns of the proximal-most stent ring, the exactly two proximal-most crowns being directly adjacent to each other.
Clause 11. A retention system comprising:
   a stent-graft including:
   a proximal body having a proximal segment including a proximal-most stent ring;
   a graft material, wherein the stent-graft has a radially compressed configuration for delivery within a vasculature and a radially expanded configuration for deployment, and
   a delivery catheter including:
      an inner shaft having a shaft lumen extending from a distal end to a proximal end of the inner shaft; and
      a distal tip coupled to the distal end of the inner shaft, the distal tip including a tip lumen and a distal-facing notch formed in a sidewall of the distal tip, wherein the tip lumen is fluidly connected to the shaft lumen to form a guidewire lumen of the delivery catheter,
   wherein the distal-facing notch is configured to receive the proximal segment of the stent-graft such that the distal-facing notch retains the proximal end of the stent-graft in the radially expanded configuration to prevent distal migration and compression of the stent-graft, and
   wherein proximal retraction of the delivery catheter releases the proximal segment from the distal-facing notch of the distal tip such that the stent-graft is no longer coupled to the delivery catheter.
Clause 12. The retention system of claim 11, wherein the proximal-most stent ring of the stent-graft includes a plurality of proximal-most crowns and wherein the proximal segment of the stent-graft to be received by the distal-facing notch is a single proximal-most crown of the proximal-most stent ring of the stent-graft.
Clause 13. The retention system of claim 11, wherein the proximal segment of the stent-graft to be received by the distal-facing notch is a loop attached to a proximal end of the stent-graft.
Clause 14. The retention system of claim 13, wherein the loop is attached to the proximal-most stent ring at the proximal end of the stent-graft.
Clause 15. The retention system of claim 13, wherein the loop is attached to the graft material at the proximal end of the stent-graft.
Clause 16. The retention system of claim 13, wherein the loop is a single loop such that the proximal end of the stent-graft includes exactly one loop.
Clause 17. The retention system of claim 13, wherein the loop has a length of about 3-20 mm.
Clause 18. The retention system of claim 13, wherein the loop is configured to constrain less than 20% of a perimeter of the stent-graft when the stent-graft is in the radially expanded configuration.
Clause 19. The retention system of claim 13, wherein the loop is coupled to a single proximal-most crown of the proximal-most stent ring or is coupled to exactly two proximal-most crowns of the proximal-most stent ring, the exactly two proximal-most crowns being directly adjacent to each other.
Clause 20. The retention system of claim 11, wherein the distal-facing notch is a protrusion formed by a slit that extends through a portion of the sidewall of the distal tip, the protrusion extending radially outward and in a distal direction towards a distal end of the distal tip.

Further disclosed herein is a proximal stent-graft retention system. The proximal stent-graft retention system includes a stent-graft having a suture loop attached to a proximal end of the stent-graft and a delivery catheter having a distal tip that includes a retention feature. The retention feature can include a slot extending through a sidewall of the distal tip and a trigger wire. The slot of the distal tip exposes a portion of the trigger wire within the tip lumen such that the trigger wire may extend through the suture loop of the stent-graft at the slot of the distal tip of the delivery catheter and is configured to retain the proximal end of the stent-graft in the radially expanded configuration via the suture loop to prevent distal migration and compression of the stent-graft. Proximal retraction of the trigger wire releases the suture loop of the stent-graft such that the stent-graft is no longer coupled to the delivery catheter.

## Claims

1. A retention system comprising:
a stent-graft including:
a proximal body having a proximal-most stent ring;
a graft material; and
a loop attached to a proximal end of the stent-graft, wherein the stent-graft has a radially compressed configuration for delivery within a vasculature and a radially expanded configuration for deployment, and
a delivery catheter including:
an inner shaft having a shaft lumen extending from a distal end to a proximal end of the inner shaft, wherein the stent-graft is disposed over a distal portion of the inner shaft in the radially compressed configuration;
a distal tip coupled to the distal end of the inner shaft, the distal tip including a tip lumen and a slot extending through a sidewall of the distal tip, wherein the tip lumen is fluidly connected to the shaft lumen to form a trigger wire lumen of the delivery catheter; and
a trigger wire extending through the trigger wire of the delivery catheter,
wherein the slot of the distal tip exposes a portion of the trigger wire within the tip lumen,
wherein the trigger wire extends through the loop of the stent-graft at the slot of the distal tip of the delivery catheter and is configured to retain the proximal end of the stent-graft in the radially expanded configuration via the loop to prevent distal migration and compression of the stent-graft, and
wherein proximal retraction of the trigger wire releases the loop of the stent-graft such that the stent-graft is no longer coupled to the delivery catheter.

2. The retention system of claim 1, wherein the stent-graft is a bifurcated stent-graft, the bifurcated stent-graft having an internal branch and an external branch that extend distally from the proximal body.

3. The retention system of any preceding claim, wherein the loop is attached to the proximal-most stent ring at the proximal end of the stent-graft; and/or wherein the loop is attached to the graft material at the proximal end of the stent-graft.

4. The retention system of any preceding claim, wherein the trigger wire is a guidewire for the delivery catheter.

5. The retention system of any preceding claim, wherein the loop is a single loop such that the proximal end of the stent-graft includes exactly one loop; and/or wherein the loop has a length of about 3-20 mm.

6. The retention system of any preceding claim, wherein the loop is configured to constrain less than 20% of a perimeter of the stent-graft when the stent-graft is in the radially expanded configuration.

7. The retention system of any preceding claim, wherein the proximal-most stent ring of the stent-graft includes a plurality of proximal-most crowns; and optionally wherein the loop is coupled to a single proximal-most crown of the proximal-most stent ring or is coupled to exactly two proximal-most crowns of the proximal-most stent ring, the exactly two proximal-most crowns being directly adj acent to each other.

8. A retention system comprising:
a stent-graft including:
a proximal body having a proximal segment including a proximal-most stent ring;
a graft material, wherein the stent-graft has a radially compressed configuration for delivery within a vasculature and a radially expanded configuration for deployment, and
a delivery catheter including:
an inner shaft having a shaft lumen extending from a distal end to a proximal end of the inner shaft; and
a distal tip coupled to the distal end of the inner shaft, the distal tip including a tip lumen and a distal-facing notch formed in a sidewall of the distal tip, wherein the tip lumen is fluidly connected to the shaft lumen to form a guidewire lumen of the delivery catheter,
wherein the distal-facing notch is configured to receive the proximal segment of the stent-graft such that the distal-facing notch retains the proximal end of the stent-graft in the radially expanded configuration to prevent distal migration and compression of the stent-graft, and
wherein proximal retraction of the delivery catheter releases the proximal segment from the distal-facing notch of the distal tip such that the stent-graft is no longer coupled to the delivery catheter.

9. The retention system of claim 8, wherein the proximal-most stent ring of the stent-graft includes a plurality of proximal-most crowns and wherein the proximal segment of the stent-graft to be received by the distal-facing notch is a single proximal-most crown of the proximal-most stent ring of the stent-graft.

10. The retention system of claim 8 or 9, wherein the proximal segment of the stent-graft to be received by the distal-facing notch is a loop attached to a proximal end of the stent-graft.

11. The retention system of claim 10, wherein the loop is attached to the proximal-most stent ring at the proximal end of the stent-graft; and/or wherein the loop is attached to the graft material at the proximal end of the stent-graft.

12. The retention system of claim 10, wherein the loop is a single loop such that the proximal end of the stent-graft includes exactly one loop; and/or wherein the loop has a length of about 3-20 mm.

13. The retention system of claim 10, wherein the loop is configured to constrain less than 20% of a perimeter of the stent-graft when the stent-graft is in the radially expanded configuration.

14. The retention system of claim 10, wherein the loop is coupled to a single proximal-most crown of the proximal-most stent ring or is coupled to exactly two proximal-most crowns of the proximal-most stent ring, the exactly two proximal-most crowns being directly adjacent to each other.

15. The retention system of any of claims 8 to 14, wherein the distal-facing notch is a protrusion formed by a slit that extends through a portion of the sidewall of the distal tip, the protrusion extending radially outward and in a distal direction towards a distal end of the distal tip.
